# EUROPEAN PATENT APPLICATION

(11) **EP 1 541 190 A1**
(43) Date of publication of application: **15.06.2005**
(21) Application number: 03388086.5
(22) Date of filing: 09.12.2003
(51) Int. Cl.: A61N 1/04, A61B 5/0408

(54) **Electromedical electrode with a detachable cable connector**

(71) Applicant: Ambu A/S, 2750 Ballerup (DK)
(72) Inventor: Nielsen, Brian, 4700 Naestved (DK); Andersen, Mette Merete, 4000 Roskilde (DK); Mortensen, Mirjam Marquard, 2750 Ballerup (DK); Vagnholm, Per, 2640 Hedehusene (DK); Petersen, Lasse Kjeld Gjoske, 2950 Vedbaek (DK); Nicolaisen, Claus, 1618 Copenhageb V (DK); Nielsen, Henrik, 4000 Roskilde (DK); Holbaek, Henrik, 3080 Tikob (DK); Jensen, Claus, 3080 Tikob (DK)
(74) Representative: Olsen, Lau Lund

(57) **Abstract**

An electromedical electrode with a detachable cable connector comprises an electrode body adapted to contact the skin of a patient. At least one flange member on the electrode body forms at least two opposed groove sections. The cable connector has two opposed edge portions slidable into the respective groove sections of the flange member, whereby the cable connector may be retained in a connected position on the electrode. The electrode body has a substantially thin configuration, the cable connector has a substantially flat engagement portion, and the at least two opposed groove sections open towards each other. Thereby the engagement portion of the cable connector, in its connected position on the electrode, is at least partly encircled by the at least one flange member.

## Description

The present invention relates to an electromedical electrode with a detachable cable connector, comprising an electrode body having a first side adapted to contact the skin of a patient and a second side opposite to the first side, at least one flange member extending along the second side of the electrode body and forming at least two opposed groove sections, the cable connector having two opposed edge portions slidable into the respective groove sections of the at least one flange member, whereby the cable connector may be retained in a connected position on the electrode.

GB 2 200 284 describes an electrode for applying a treatment current to the skin of a person, especially an electrode that is soaked in water or medicament solution when in use. In one embodiment of the electrode, a peripheral separating wall is provided around a circular nonconductive base plate, whereby a cavity is formed, in which a conductive medium may be received. The lower side of the cavity is covered by means of a plate having a plurality of holes, through which the conductive medium may soak a conductive solution and subsequently contact the skin. On the top side of the electrode, on the side of the base plate opposite the cavity, there is provided to spaced guide rails. A box-shaped hold member has an open bottom side and has, along the lower edges of two opposed sidewalls, respectively, two edge portions which are bent towards each other and are slidable onto the guide rails in order to mount the hold member on the electrode. The inside of the box-shaped hold member is provided with a conductive leaf spring, which elastically may slide over a metal projection on the top side of the electrode and thereby retain the hold member on the electrode in use. The conductive leaf spring arrangement is provided with a cable for electrically connection of the electrode with a treatment apparatus. The box-shaped hold member may be disconnected from the electrode by lifting the leaf spring out of engagement with the metal projection on the electrode and sliding the hold member along the guide rails. During treatment, the electrode is maintained in close contact with the patient's skin by grabbing around the box-shaped hold member. Obviously, this arrangement is not suitable for the continuous monitoring of, for instance, the heart rate pulse of a patient, because the electrode, even if it were provided with an adhesive, would easily be ripped off the skin of the patient as a result of its bulky configuration that makes it stand out from the skin. Apart from the considerable risk that the electrode is ripped off the skin, for instance when the patient turns around when lying in a bed, the electrode will also result in a most uncomfortable situation, if the patient is lying on top of the electrode, whereby its bulky configuration will cause it to depress the skin of the patient. This is of special importance and is totally unacceptable in the case of neonatals, because their skin is highly susceptible to damages.

US 4,090,760 discloses a tunnel-shaped contact member that is attached on the top side of a body-applied electrode for medical equipment. The tunnel-shaped contact member has a rectangular tunnel formed by top and bottom planar members separated by side support pillars. At opposed sides of the rectangular tunnel, next to the support pillars, electrically conductive contact pillars are arranged, which are electrically connected to the electrode. A cable connector has the shape of a horseshoe with a base portion, where a cable is connected, and two arms being elastically bendable against each other and having wedge-shaped end surfaces which may slide along the contact pillars by insertion of the cable connector into the tunnel of the contact member, whereby the arms are bent towards each other. Near its free end, each arm is provided with a notch so that the notches open away from each other and may engage the contact pillars of the contact member by elastic flection of the two arms away from each other. In the bottom of each notch is arranged an electrically conductive contact member which establish the electrical connection between the cable and the contact pillars of the electrode. However, this electrode arrangement is inherently also very bulky and consequently has the same disadvantages as the above-mentioned electrode.

US 5,431,166 describes a medical electrode having a low profile. The electrode includes a base sheet constructed of a flexible conductive polymer, whereby a tab is laminated to the top of the base sheet to form a pocket between the tab and the base sheet extending along the base sheet for receiving a hook connector. The hook connector has a bill and shank joined by a bend, the bill fitting between the pocket and the shank lying on top of the tab when the bill is so engaged to allow a low profile connection to the electrode. However, this electrode has the disadvantage that activity of the patient in many situations will cause the hook connector to slide out of engagement with the pocket of the electrode. In order to overcome this disadvantage, in one embodiment of the hook connector, the connector is provided with barbs extending on either side of the bill. The barbs allow insertion of the bill into the pocket with relative little resistance but than prevent disengagement of the bill from the pocket, in the manner of a barb on a fish hook, and prevent the connector from being removed from the electrode without destruction of the tab. However, this hook connector is obviously not suitable for repetitive connection and disconnection with the electrode, because the tab is destroyed after the first use. Consequently, this electrode arrangement is not suitable in the monitoring of neonatals, whereby the electrode is permanently adhered to the skin of the neonatal, but the cable connector is only connected to the electrode periodically during monitoring and disconnected from the electrode between monitoring in order to free the neonatal from the cables of the monitoring system.

US 4,401,356 describes an electrical terminal for making conductive connection with a printed circuit conductor on one side of a dielectric sheet of an electrode comprises a wiper section adapted to make contact with the printed circuit conductor and a blade section adapted to make contact with the opposite side of the sheet, the wiper section being struck from the blade section but leaving a part of the wiper section within the perimeter of the strike out opening so that upon insertion of the sheet between the blade and wiper sections, those sections spread apart and apply opposed clamping pressure to opposite sides of the sheet. In one embodiment, the blade portion is provided with a tine which projects through an opening in the dielectric sheet in the engagement position of the electrical terminal, thereby inhibiting relative sliding movement of the sheet and the terminal. After the terminal is assembled to the dielectric sheet a covering foam sheet is fixed on top of the terminal on the electrode in order to provide a comfortable back-pad construction for the patient. However, the procedure of connecting the terminal with the electrode of this arrangement is rather complicated and therefore not suitable for repetitive connection and disconnection operations which are usual, for instance in the treatment of neonatals.

US 6,418,347 discloses a transcutaneous medical electrode including af flexible electrically conductive sheet having an electrically conductive adhesive layer on one surface. A female connector having an opening for receiving a conventional lead wire tip is secured to the opposite surface of the sheet. A sheathed male connector includes a tip for insertion into the female connector opening without exposing the tip to touching by the operator of the device or the patient. The female connector is preferably tubular with an approximately axial opening. The female connector may be secured to the sheet, parallel to the sheet, with the male connector extending beyond and edge of the sheet or alternatively, the female connector may be secured to the sheet in a bent arrangement with the male connector extending away from the sheet. However, the female connector and the male connector extending from the back of the electrode may cause the electrode to be torn off the skin by activity of the patient and will furthermore depress the skin, if the patient is lying in a bed on top of the electrode.

US 4,632,121 shows different embodiments of electrodes having a male or female connector for connection with a corresponding male or female connector on a cable in similar manner as the above-mentioned transcutaneous electrode. Due to the bulky configuration of the male and female connectors, these electrodes have the same disadvantages as mentioned above.

US 4,126,126 discloses another example of an electrode having a female connector corresponding to a male connector of a cable. This electrode has the same disadvantages as the above-mentioned electrodes of similar type.

The object of the present invention is to provide an electromedical electrode that is more gentle to the skin of a patient than known electrodes, and whereby the cable connector easily may be attached to and detached from the electrode body several times.

In view of this object, the electrode body has a substantially thin configuration, the cable connector has a substantially flat engagement portion, the at least two opposed groove sections open towards each other, whereby the engagement portion of the cable connector, in the connected position of the cable connector on the electrode, is at least partly encircled by the at least one flange member.

By providing a flat engagement portion of the cable connector which along part of its periphery may engage a groove provided in a flange member, an extremely slim configuration of the arrangement of the electrode body and the cable connector may be obtained, because the flat engagement portion of the cable connector may lie flat on the second side of the electrode body, in the connected position of the cable connector on the electrode. As the flat engagement portion is at least partly encircled by the flange member, a very stable connection between the cable connector and the electrode body may be obtained. Furthermore, by adjusting the tolerances between the groove and the edge portions of the cable connector, a suitable friction force between these two members may be obtained so that the engagement portion may be retained reliably in its connected position on the electrode. Because the engagement portion, in its engaged position, is partly encircled by the at least one flange member and thereby located between the at least two opposed groove sections, it may be freely accessible to be slid out of engagement by a fingertip, if necessary.

In an embodiment, a cable extends from the cable connector in such a direction that, by pulling the cable, the engagement portion may be slid in a mounting direction into the groove sections of the at least one flange member, until it reaches its connected position on the electrode, and, in its connected position, the engagement portion is prevented by means of the flange member from sliding further in the mounting direction. Thereby the mounting of the cable connector on the electrode body is facilitated, and it is prevented that the connector by subsequently pulling the cable accidentally is disconnected from the electrode body.

In an embodiment, in the connected position of the cable connector on the electrode, a cable extends from the cable connector in a direction forming an angle with the second side of the electrode body of at least 8 degrees, and preferably at least 12 degrees. Thereby a slim configuration of the arrangement of the electrode body and the cable connector is further facilitated, and the cable may project from the cable connector close to the second side of the electrode body.

In an embodiment, in the connected position of the cable connector on the electrode, a free top side of the engagement portion of the cable connector is substantially flush with a free top side of the at least one flange member. Thereby a very integrated configuration of the cable connector and the flange member is obtained in the connected state, whereby protruding edges are avoided that could otherwise get caught incidentally by activity of the patient and thereby cause the engagement portion to be disengaged from the flange member. Furthermore, as a result of the integrated configuration of the engagement portion and the flange member, the necessary amount of material for these two members may be distributed in a most effective way, in terms of obtaining an overall slim configuration of the electrode. Additionally, an appealing smooth finish of the electrode is obtained, thereby influencing its total appearance positively.

In another embodiment, the two opposed edge portions of the cable connector are depressed in relation to the free top side of the engagement portion of the cable connector. This feature further improves the integrated configuration of the engagement portion and the flange member. Furthermore, the depressed edge portions of the cable connector provides a shoulder on the cable connector which may abut an edge of the flange member and thereby provide a further contact surface between the cable connector and the flange member, thereby ensuring an even more stable engagement between the to parts.

In another embodiment, the at least one flange member is constituted by a socket having substantially the shape of part of a ring, the at least two opposed groove sections being formed by opposed parts of the socket, respectively. This configuration of the flange member as a single element in the form of a socket provides a robust and simple construction which is easy to manufacture, for instance by injection moulding. As the socket has substantially the shape of a part of a ring, the exact orientation of the engagement portion, at the beginning of its insertion into the socket, is not essential, as it may be slightly rotated during its insertion to be positioned appropriately in the socket when it has been fully inserted.

In another embodiment, the socket has substantially the shape of a circular section of at least 180 degrees, preferably at least 200 degrees, and in particular at least 220 degrees of a ring. In this way it is ensured that the engagement portion of the cable connector is encircled in a sufficient degree by the socket in order to obtain the most efficient engagement between the connector and the socket.

In another embodiment, the groove sections have a substantially U-shaped cross-section delimited partly by a channel formed in the socket and having a substantially L-shaped cross-section and partly by the second side of the electrode body. Thereby it is ensured that the engagement portion, in its engaged position, is positioned as close to the electrode body as possible, whereby a very slim electrode configuration may be obtained. Furthermore, the second side of the electrode body may be elastically deformable by insertion of the engagement portion, thereby providing a frictional force between the engagement portion and the channel formed in the socket whereby the engagement portion is retained securely in its engaged position in the socket.

In another embodiment, the at least two groove sections are arranged symmetrically on either side of a projection formed integrally in the socket and separating the groove sections from each other, and an indentation is formed between the two opposed edge portions of the cable connector, the indentation receiving the projection of the socket in the connected ) position of the cable connector on the electrode. The indentation in the cable connector and the corresponding projection of the socket ensures correct orientation of the cable connector in the socket in its engaged position. This is in particular advantageous when the socket has a circular configuration whereby the cable connector otherwise could by rotatable in the socket. Furthermore, a better engagement between the cable connector and the socket is achieved.

In another embodiment, each of the two opposed edge portions of the cable connector has at least one notch in which a tooth formed in the channel in the socket may engage elastically, when the cable connector is inserted in the socket. The engagement between the tooth and the notch may provide an appropriate retaining force to keep the cable connector in its engaged position. The retaining force thereby achieved may be in addition to a frictional force between the engagement portion and the socket.

In another embodiment, a recess is formed in the free top side of the socket opposite the projection that separates the groove sections in the socket, and the recess, in the connected position of the cable connector on the electrode, receives part of a cable extending from the cable connector. In this way, the cable may extend, at least partially, from a side wall of the engagement portion and thereby rather close to the second side of the electrode body. Thereby, the overall height of the electrode is kept at a minimum.

In a structurally advantageous embodiment, an electrically conductive sensor element of the electrode body is exposed on the second side of the electrode body, and at least one contact element of the cable connector contacts the sensor element in the connected position of the cable connector on the electrode. In this way good electrical contact may be established between the cable connector and the electrode body when the engagement portion lies flat against the second side of the electrode body, thereby providing a slim configuration.

In another embodiment, the sensor element is sandwiched between an electrically nonconductive electrode foil and an electrically conductive and adhesive gel, the nonconductive electrode foil having, in an area encircled at least partly by the socket, at least one aperture corresponding to the at least one contact element of the cable connector. The nonconductive electrode foil prevents the patient from touching the electrically conductive sensor element whereby the measurements performed by means of the electrode could otherwise be disturbed.

In another embodiment, the at least one aperture in the nonconductive electrode foil comprises two opposed elongated apertures, each extending along a respective one of the two opposed groove sections formed by the socket. As the edge portions of the engagement portion are pressed against the second side of the electrode body by engagement with the groove sections, a good contact between the contact elements of the cable connector and the sensor element is obtained along each of the two opposed groove sections.

In another embodiment, the electrically conductive sensor element is formed by an electrically nonconductive sensor foil having a first side with a first electrically conductive coating contacting the electrically conductive and adhesive gel and a second side with a second electrically conductive coating, the first electrically conductive coating covering substantially the entire area of the first side of the nonconductive sensor foil, and the second electrically conductive coating covering the areas of the conductive sensor element exposed through the at least one aperture in the nonconductive electrode foil. In this way, the electric current may be distributed appropriately over the area of the electrically conductive gel by the application of a minimum quantity of the material providing the electrically conductive coatings. This in turn provides good X-ray translucency, which enables X-ray examination of the patient during the application of the electrode to the skin of the patient.

In another embodiment, the electrically conductive sensor element has an edge region extending along the entire peripheral edge of the sensor foil, whereby said edge region is not covered by the first and second electrically conductive coatings, and a part of said edge region is coated with conductive ink.

By leaving the edge of the sensor element free from the electrically conductive coatings, edge effects, such as effects promoted by the presence of oxygen, may be reduced. The conductive ink may be a material different from the conductive coatings and less susceptible to said edge effects and may thereby provide the electrical connection between the first and the second electrically conductive coatings. The use of conductive ink also permits good X-ray translucency in the edge region.

In another embodiment, the first electrically conductive coating comprises silver and silver chloride, the second electrically conductive coating comprises silver and the conductive ink comprises carbon. The coating comprising silver and silver chloride may provide a conductivity in the interface between the first coating and the conductive gel and the second electrically conductive coating comprising silver in combination with the conductive ink comprising carbon may provide the electrical connection from the first coating to the cable connector by the application of materials which are more economical in use.

In another embodiment, the sensor element is arranged at a central part of the electrode body. In this way, a good distribution of the electrical current to the electrically conductive gel is obtained without applying the sensor element over the entire area of the electrode body.

In an embodiment easy to manufacture, the socket has legs extending through corresponding holes in the electrically nonconductive electrode foil and the sensor element, an end of each leg having expanded cross-section and thereby securing the socket on the electrode body. Thereby a good mechanical connection between the socket and the electrode body is obtained in a simple manner.

In another embodiment, the socket and its legs are integral, and the expanded cross-section of each leg is formed by heat-melting the plastic material of the leg. Thereby the socket and its legs may be injection moulded as one unit and connected to the electrode body in a simple manner.

In another embodiment, the cable is insert moulded in the engagement portion of the cable connector, at least one cavity is formed in a bottom side of the cable connector, whereby a conductor of the cable is exposed in the cavity, and the at least one contact element of the cable connector is formed by a coating of conductive ink extending to the exposed cable conductor and thereby establishing electrical connection to the latter. Thereby the cable connector may be formed directly onto an end of the cable in one moulding operation. Subsequently the cable connector only has to be coated with the conductive ink and is then ready for use.

In another embodiment, a pointed notch is formed in the top side of the engagement portion opposite each cavity in the bottom side of the engagement portion. The pointed notch may be the result of a projection formed in the mould, in which the cable connector is injection moulded, whereby the projection may keep the cable conductors in position next to the cavity to be formed in the bottom side of the cable connector, during the injection moulding, whereby it is ensured that the conductors of the cable are exposed in the cavity after the moulding.

In an alternative embodiment, the engagement portion of the cable connector comprises two corresponding parts which are joined together so that a cable end is secured between said two parts. The two corresponding parts may be injection moulded, possibly connected by means of a film hinge, and subsequently joined together in an assembling operation.

In another alternative embodiment, the engagement portion of the cable connector has the form of a ring adapted to deform elastically, when inserted into the at least one flange member, thereby providing a frictional force between the ring and the flange member. The ring may be manufactured from a rather stiff material and deformed elastically by changing its entire shape slightly, for instance changing from a circular into a slightly oval shape.

In another embodiment, the ring has a substantially rectangular shape having two opposed long sides constituting the edge portions slidable into the respective groove sections of the at least one flange member, and said edge portions are slightly curved symmetrically in relation to each other. The slightly curved edge portions may then be deformed towards each other by insertion of the cable connector in the socket, thereby providing the frictional force necessary for the retainment of the connector.

In a further alternative embodiment, the engagement portion of the cable connector and the at least one flange member on the electrode body are made of electrically conductive plastic, such as polypropylene comprising carbon fibres, and preferably at least the top side of the engagement portion and the top side of the cable connector are provided with an electrically nonconductive coating. Thereby the cable connector may be injection moulded onto an end of the cable in one simple moulding operation, after which only the top side has to be provided with an electrically insulating coating.

In another embodiment, the engagement portion of the cable connector is formed integrally with a slim grip portion extending opposite the cable, the grip portion bending slightly upward in relation to the top side of the engagement portion and preferably being perforated by a cutout. The grip portion facilitates in particular the operation of detaching the cable connector from the electrode body.

In another embodiment, the electrode body has a substantially circular periphery provided with two or more radially extending cuttings, whereby each cutting, along substantially its entire length, has a minimum width of at least 5 per cent, and preferably at least 6 per cent, of the diameter of the electrode body, and the at least one flange member is arranged centrally on the electrode body. By providing such cuttings, having a relatively large width compared to the diameter of the electrode body, the first side of the electrode body may adapt and adhere to the skin of a patient, even in such situations where the skin is very uneven.

In another embodiment, each cutting has opposed side edges forming an angle of at least 6 degrees, preferably at least 8 degrees, and more preferably at least 9 degrees, these side edges are joined by a circular arc and join the circular periphery of the electrode body via circular arcs. This contour of the electrode body provides good adaptation to uneven skin, especially by small electrode sizes as used for neonatals.

In another embodiment, the general thickness of the electrode measured between the free top side of the at least one flange member and the first side of the electrode body is smaller than 3 millimetres, preferably smaller than 2 millimetres, and more preferably approximately 1.8 millimetres. Thereby a very slim electrode is obtained which permits a very gentle monitoring of neonatals.

The invention will now be explained in more detail below by means of examples of embodiments with reference to the schematic drawing, in which
Fig. 1 is a perspective view of an electromedical electrode with a cable connector according to the invention,

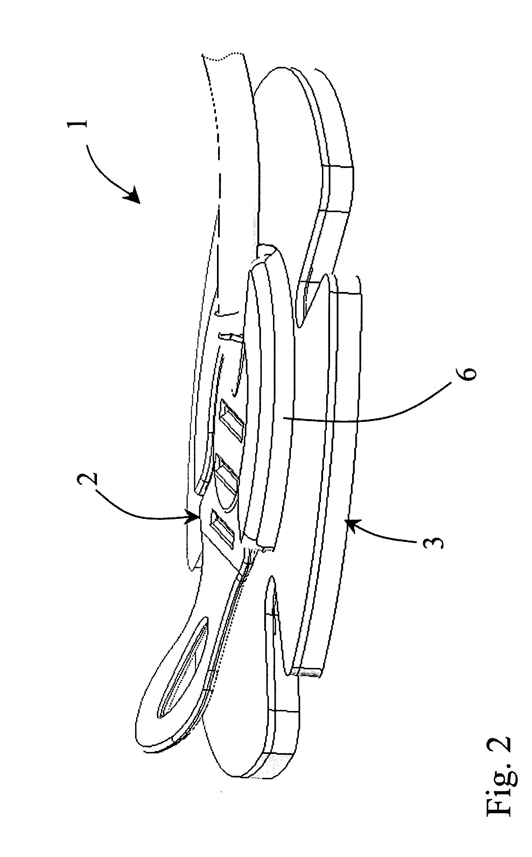

Fig. 2 is another perspective view of the electrode in Fig. 1, seen from a different angle,
Fig. 3 is a perspective, partly sectional view of the electrode in Fig. 1,
Fig. 4 is a sectional view along a diameter of the electrode in Fig. 1,
Fig. 5 and 6 are a top view and a side view, respectively, of the electrode in Fig. 1,
Fig. 7 is an exploded view of the electrode in Fig. 1,

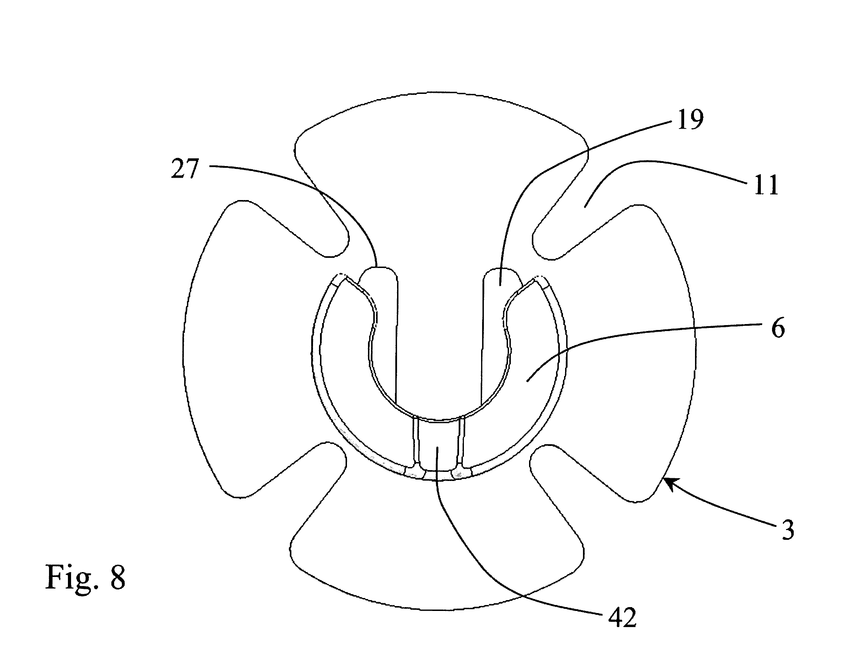

Fig. 8 is a top view of the electrode body according to the invention, without the corresponding cable connector,

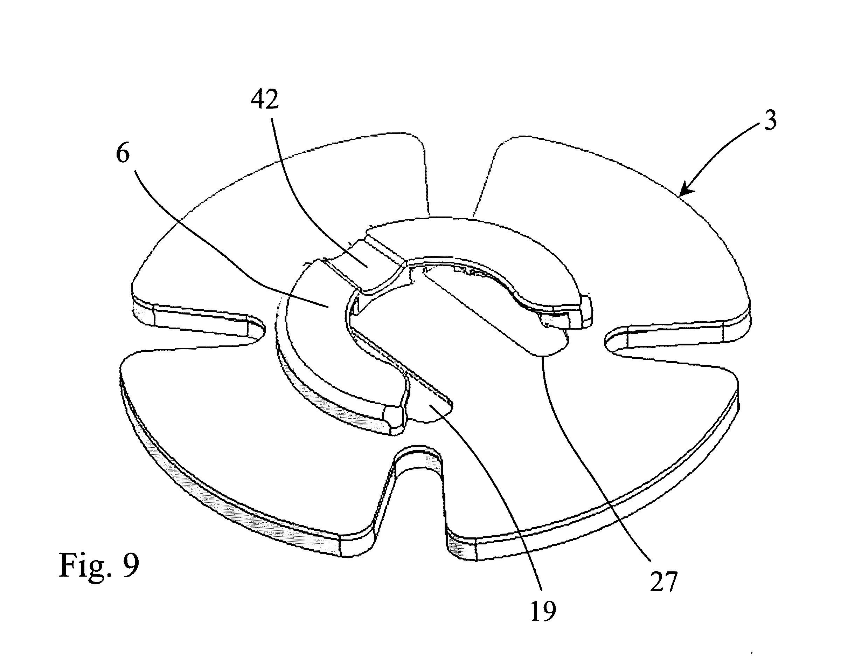

Fig. 9 is a perspective view of the electrode body in Fig. 8,
Fig. 10 is a perspective view of the socket of the electrode of Fig. 1, seen partly from above,
Fig. 11 is a bottom view of the socket in Fig. 10,
Fig. 12 is a perspective view of the socket in Fig. 10, seen partly from below,
Fig. 13 is a perspective view of the socket in Fig. 10, seen in the direction of the arrow in Fig. 10,
Fig. 14 is a perspective view of the cable connector of the electrode in Fig. 1, seen partly from above,
Fig. 15 is an end view of the cable connector in Fig. 14, seen from the cable side,
Fig. 16 is a sectional view along the line XVI-XVI of Fig. 17,
Fig. 17 is a side view of the cable connector in Fig. 14,
Fig. 18 is a longitudinal sectional view of the cable connector in Fig. 14,
Fig. 19 is a top view of the sensor element of the electrode, as seen in perspective view in Fig. 7,
Fig. 20 is a perspective view corresponding to Fig. 9 of another embodiment of the electrode body,
Fig. 21 is a perspective view of another embodiment of the electrode in Fig. 1,
Fig. 22 is a perspective, partly sectional view of the embodiment in Fig. 21,
Fig. 23 is a perspective view of the electrode body of the embodiment of the electrode in Fig. 21, seen partly from above,
Fig. 24 is a perspective, sectional view along a diameter of the embodiment of the electrode in Fig. 21.
Fig. 25 is a sectional view along a diameter of another embodiment of the electrode,
Fig. 26 is a top view of another embodiment of the electrode, and
Fig. 27 is a top view of yet another embodiment of the electrode.

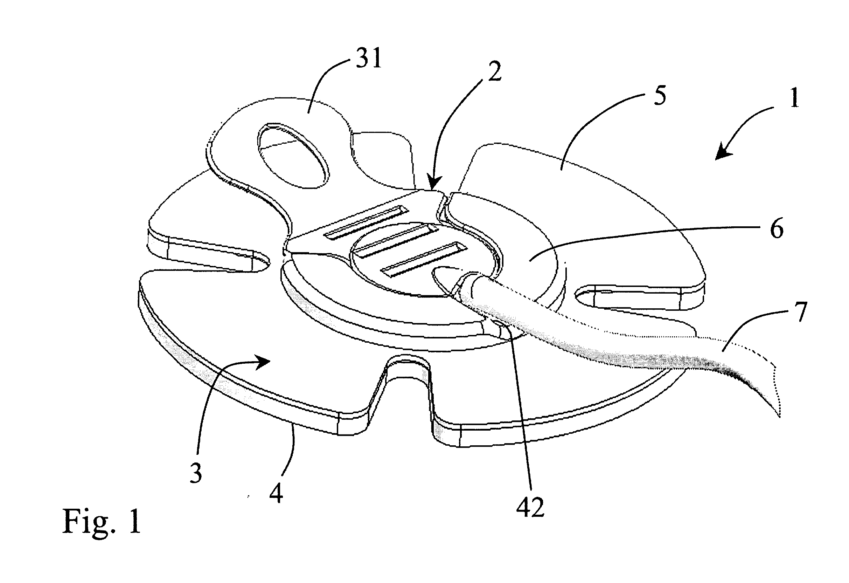

Fig. 1 shows an electromedical electrode 1 with a detachable cable connector 2 according to the invention. The electrode arrangement shown in Fig. 1 is particularly advantageous as a neonatal electrode, because the construction permits the electrode to be produced with very small overall dimensions, and at the same time the electrode is easy to handle even in the treatment and monitoring of neonatals. However, the electrode 1 according to the invention is equally applicable in any other situation, whereby skin electrodes are employed to establish electrical contact between the skin of a patient and a measuring or treating apparatus. Such electrodes are employed for measuring electrical signals generated in or by the body. For instance, the functions of the heart may be registered by recording the signals of the heart on an electrocardiogram (ECG).

The skin electrode in Fig. 1 comprises a substantially thin electrode body 3 having a first side 4 adapted to contact the skin of a patient and a second side 5, which is the top side when the electrode is orientated as shown in Fig. 1, and on which is mounted a central socket 6 having substantially the shape of a circular section of 2/3 of a ring and in which the cable connector 2 is detachably retained. The cable connector 2 is provided with an electric cable 7 to be connected with the not shown measuring or treating apparatus for establishing the electrical contact between said apparatus and the skin of the patient.

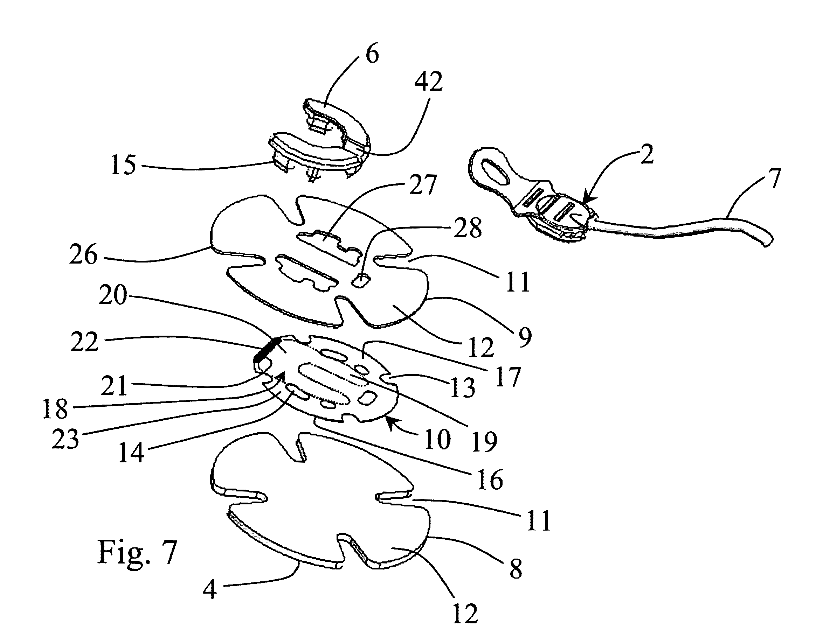

As shown in Fig. 7, the electrode body 3 comprises a bottom layer 8 containing an electrically conductive and adhesive gel, which forms the first side 4 of the electrode body 3, a top layer 9 formed of an electrically nonconductive electrode foil, an electrically conductive sensor element 10 sandwiched between the bottom layer 8 and the top layer 9, and a socket 6 which is mounted on top of the three sandwiched layers.

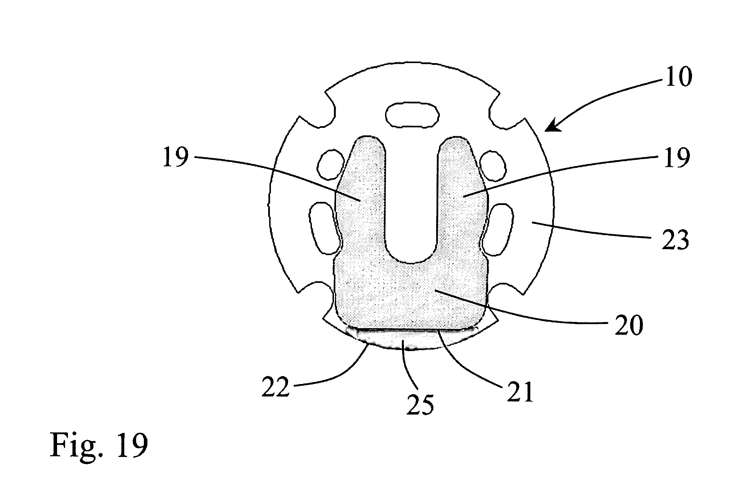

The bottom and top layers 8, 9 of the electrode body 3 has corresponding peripheral shapes which, when sandwiched together, form a peripheral shape of the electrode body 3 in the form of a four-leaf clover having radially extending cuttings 11 between each of its leaves 12, which be explained in more detail below. The sensor element 10 comprises a core layer in the form of a sensor foil 23 made of a polyethylene foil having a thickness of 0.05 millimetres and having a substantially circular periphery with a diameter smaller than the diameter of the periphery of the bottom layer 8 and having smaller cuttings 13 corresponding to the cuttings 11 of the bottom layer 8 and the top layer 9. Furthermore, the sensor element 10 have five holes 14 for the insertion of legs 15 of the socket 6. A first side 16 of the sensor element 10 comprises over substantially the entire area of the sensor foil 23 a (not shown) first electrically conductive coating comprising silver and silver chloride in order to establish an efficient low impedance electrical contact between the sensor element 10 and the electrically conductive and adhesive gel of the lower layer 8 of the electrode body 3. A second side 17 opposite the first side 16 of the sensor element 10 comprises a second electrically conductive coating 18 composed of silver and located in two spaced, longitudinally extending areas 19 in an area of the electrode body 3 that is encircled by the socket 6 in the mounted state of the electrode body 3. The second electrically conductive coating 18 further comprises an area 20 that joins the two longitudinally extending areas 19 and is terminated along a line 21 located at a distance of approximately 0.5 millimetres to 1 millimetre from an edge 22 of the sensor foil 23, see also Fig. 19, in order to avoid edge effects, for instance resulting from the presence of oxygen.

In order to establish electrical connection between the first electrically conductive coating and the second electrically conductive coating 18 of the sensor element 10, a small area 25 extending around an edge from the first side 16 to the second side 17 of the sensor foil 23 is provided with conductive ink, thereby establishing the necessary electrical connection. The conductive ink may comprise carbon or any other suitable material in order to provide the electrical conductivity, and it may for instance be applied to the edge of the sensor element by means of pad printing. Alternatively to the application of the conductive ink, the sensor element 10 may be penetrated by a rivet that provides the electrical connection between the first electrically conductive coating and the second electrically conductive coating 18. It is to be noted that although substantially the entire area of the first side 16 of the sensor foil 23 is covered by the first electrically conductive coating, preferably said coating terminates at a distance of approximately 0.5 millimetres to 1.0 millimetre from the periphery of the sensor foil 23 in order to avoid edge effects, for instance resulting from the presence of oxygen, as also explained above.

As a further alternative, the sensor foil 23 itself may be made electrically conductive, for instance by suspending conductive particles in the foil material, such as suspending carbon in a polyvinyl chloride foil. Thereby the sensor foil 23 itself will provide the electrical connection between the first electrically conductive coating and the second electrically conductive coating 18.

An advantage of employing materials such as foils made conductive by means of suspended conductive particles and electrically conductive coatings and inks instead of, for instance, metallic foils in order to provide electrical contact is that a good X-ray translucency is obtained, whereby a patient may be X-ray examined during the application of the electrode to the skin. During the X-ray examination, for instance an electrically conductive coating may be recognized by the physician by its contour; however, it is also possible to see through the conductive coating and thereby recognize structures of the patient's body situated thereunder. Due to the special contour of the electrically conductive foils and/or electrically conductive coatings used in the electrode 1 according to the invention, these are during X-ray examination easily recognized by the physician as being parts of the electrode and not for instance pertaining to the patients body.

The top layer 9 of the electrode body 3 is formed of a non-electrically conductive electrode foil in textile having a thickness of 0.2 millimetres and being coated with a suitable adhesive on a lower side 26 that contacts partially the sensor element 10 and the gel 8 in the assembled state of the electrode body 3. The top layer 9 is provided with two apertures 27, each covering an area corresponding to one of the longitudinally extending areas 19 of the second coating 18 of the sensor element 10 and two of the holes 14 situated next to said area 19 in the sensor element 10, and a third aperture 28 corresponding to one of the holes 14 situated in the sensor element 10 between the ends of the two areas 19 of the second coating 18.

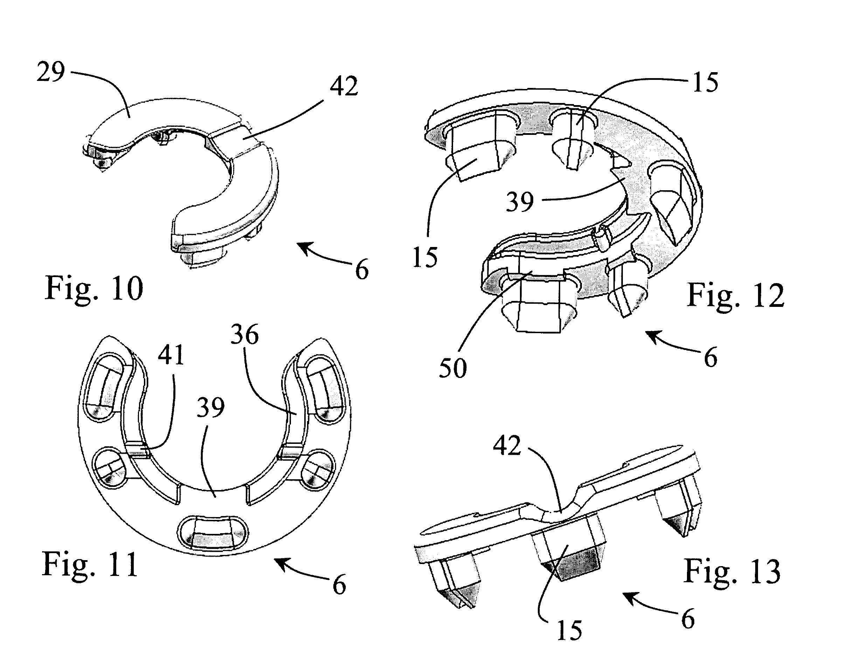

Referring to the Figures 10 to 13, the socket 6 has a basis 29 having substantially the shape of 2/3 of a ring and being provided on a lower side with five protruding legs 15 corresponding to the five holes 14 in the sensor element 10. The socket 6 is injection moulded as an integral element of plastic material. When assembling the electrode body 3, firstly the sensor element 10 is adhered to the lower side 26 of the top layer 9 of the electrode body, and subsequently the socket 6 is positioned on the top side of the top layer 9 with its legs 15 extending through the apertures 27, 28 of the top layer 9 and the apertures 14 of the sensor element 10. By means of ultrasound, the ends of the legs 15 protruding from the first side of the sensor element 10 are expanded and flattened on said first side of the sensor element, whereby the socket is locked permanently to the sensor element 10 and the top layer 9 of the electrode body. During the mounting of the socket 6, small protrusions 50 on the bottom side of the socket keeps the sensor element 10 in flat condition. Finally, the bottom layer 8 of gel is adhered to the lower side of the combination of the top layer 9 and the sensor element 10.

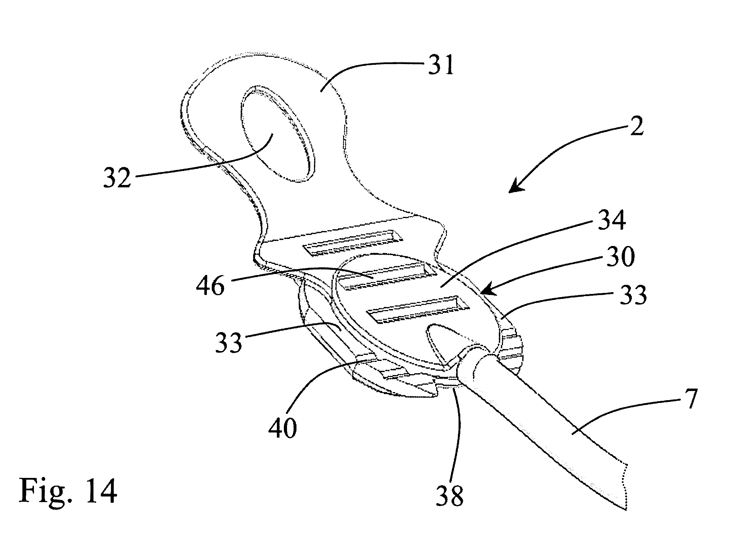

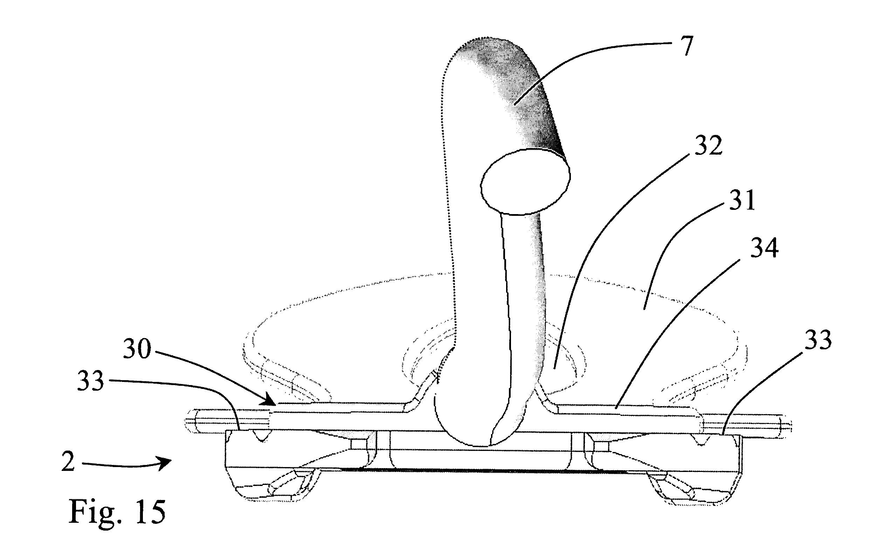

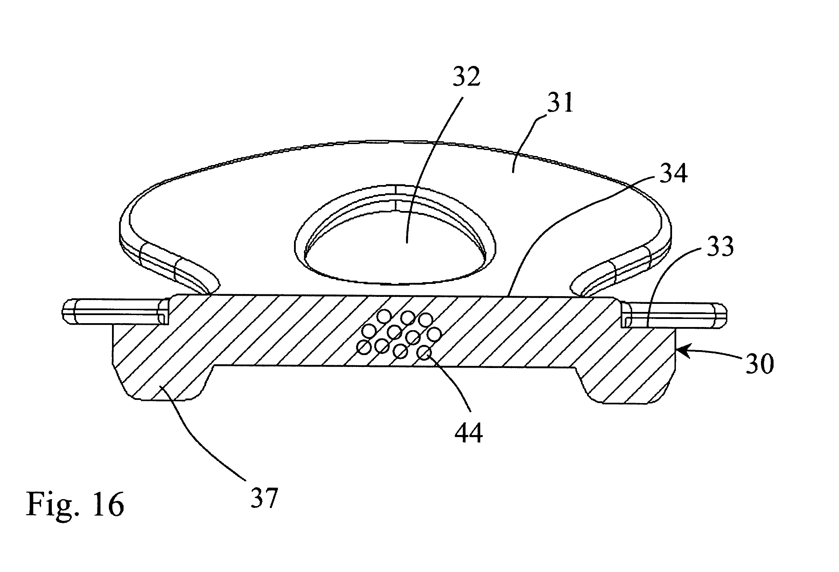

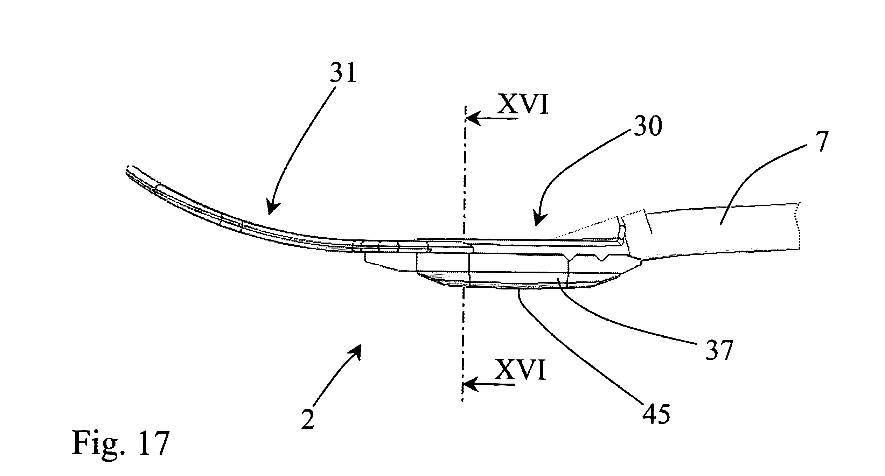

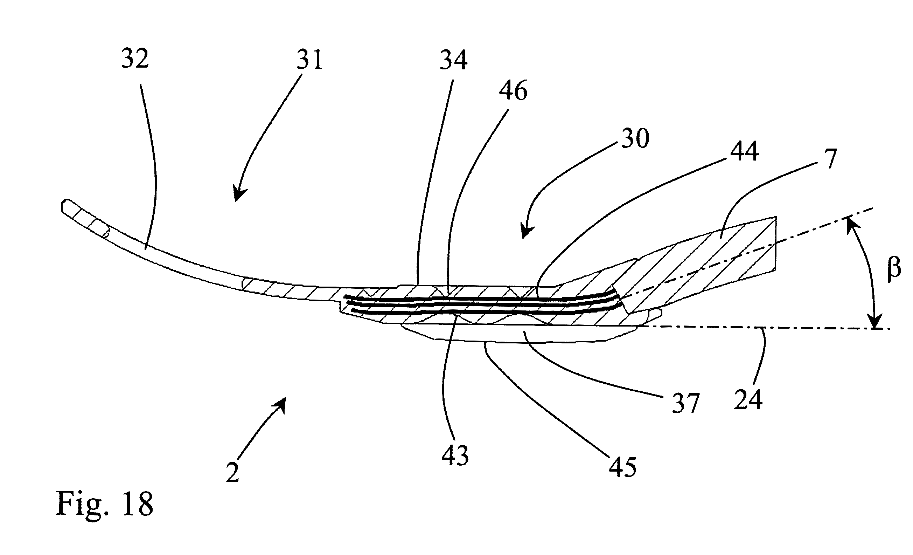

Referring now to Figures 14 to 18, the cable connector 2 is composed of a substantially thin and flat engagement portion 30, a first end of which is provided with the cable 7 extending therefrom, and a second end of which is provided with a slim grip portion 31 extending in a direction opposite the cable and bending slightly upward in relation to the engagement portion, see Figs. 17 and 18. The grip portion 31 facilitates the handling of the cable connector, especially when sliding the engagement portion 30 out of the socket 6. To this end, the grip portion 31 is further provided with an oval cutout 32 in which a fingertip may rest. The engagement portion 30 of the cable connector is preferably slid into the socket by drawing it by the cable 7 by means of two fingertips.

The grip portion 31 may have a number of other configurations than those shown with a view to enable easy gripping and disconnection of the cable connector and at the same time providing an electrode 1 having an overall slim configuration in order to ensure gentle handling of the skin of the patient. As an example, the grip portion 31 may be a piece of thin foil or textile that is insert moulded in the engagement portion 30 and extends therefrom, thereby lying flat against the second side 5 of the electrode body 3 in the connected position of the cable connector on the electrode. Such thin foil provides for a very slim electrode configuration and is easy to lift up and grip. Similarly, the grip portion 31 may be injection moulded integrally with the engagement portion, but connected to this by means of a film hinge. Furthermore, the grip portion 31 may be a piece of thread extending from the engagement portion; it may even be an end of the cable 7.

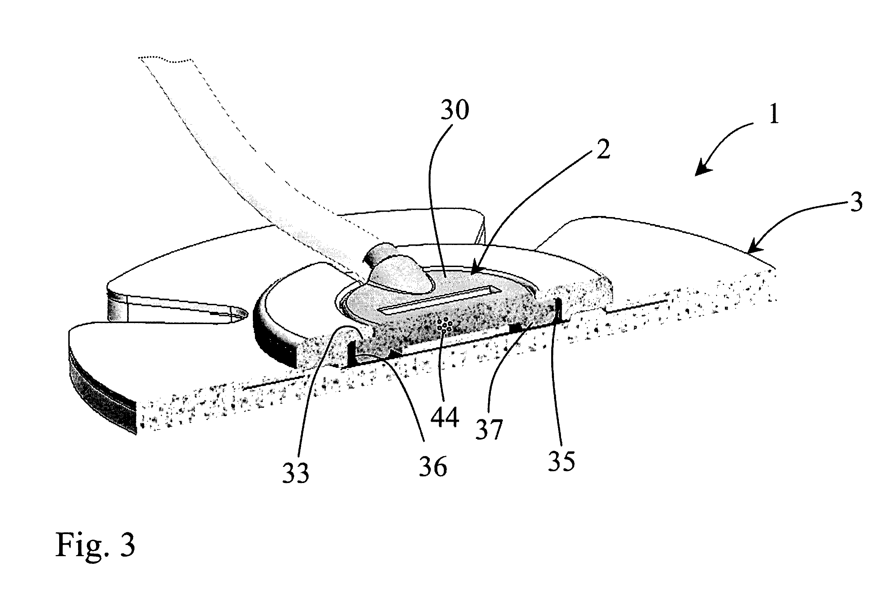

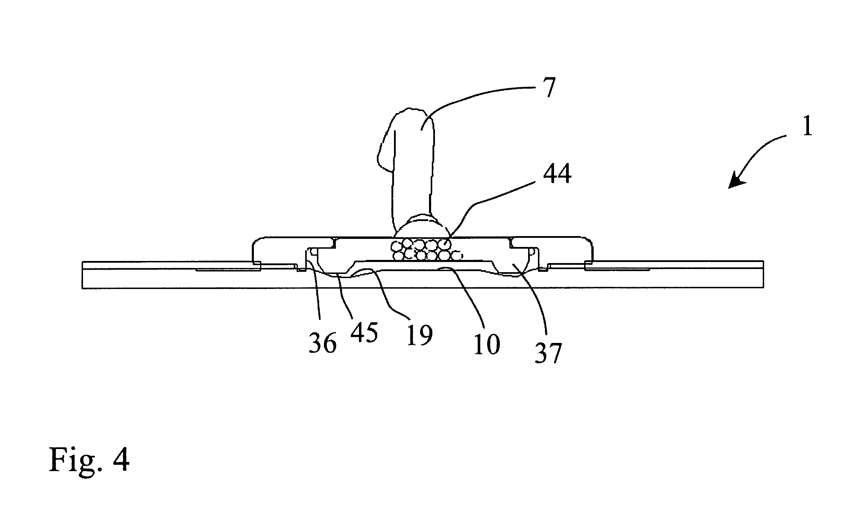

Referring to Figures 14 to 16, the engagement portion 30 has two opposed edge portions 33 which are depressed in relation to a top side 34 having a substantially oval periphery. In the connected state of the cable connector on the electrode body, see Figures 3 and 4, the depressed edge portions 33 of the engagement portion 30 are received in two opposed groove sections 35 opening against each other and being delimited partly by a channel 36 formed in the bottom side of the socket 6 and having L-shaped cross section and partly by the longitudinally extending areas 19 of the second electrically conductive coating 18 of the sensor element 10, which is exposed through the apertures 27 of the top layer 9 of the electrode body 3. Below each depressed edge portion 33, the engagement portion 30 is provided with a downward protruding rib 37 extending along the depressed edge portion 33, also received in a respective groove section 35 and contacting the electrically conductive area 19 of the sensor element 10 in the engaged position of the cable connector on the electrode, as shown in Figs. 3 and 4.

As it may be seen in Fig. 14, an indentation 38 is formed between the two depressed edge portions 33, located below the point where the cable 7 extends from the engagement portion 30, said indentation 38 receiving a projection 39 formed in the socket 6 and separating two opposed sections of the L-shaped channel 36, see Figures 11 and 12, thereby ensuring correct positioning of the engagement portion 30 in the socket 6. Furthermore, each edge portion 33 has a notch 40 in which a tooth 41 formed in the corresponding section of the L-shaped channel 36 in the socket 6 may engage elastically, thereby keeping the engagement portion 30 in place in the socket 6 after its insertion. It should be noted, that in Fig. 14 each edge portion 33 is provided with two notches 40 in order to enable insertion in a socket having two teeth 41 in either one of the two opposed channel sections 36, although the embodiment showed in Fig. 11 has only one tooth 41 on either side. As it may be seen in Fig. 1 and 10, the socket 6 has a recess 42 in its top side, located over the projection 39, in order to take up part of the cable 7 in the inserted position of the engagement portion in the socket.

Referring now to Fig. 18, the engagement portion 30 of the cable connector is provided with two cavities 43 in a bottom side of the engagement portion, in the centre of which cavities 43 conductors 44 of the cable 7 are exposed. The cavities 43 and the two ribs 37 are provided with an integrated coating of conductive ink whereby contact elements 45 on the ribs 37 are established in order to establish the electrically contact between the cable 7 and the electrically conductive sensor element 10 in the electrode body. Fig. 4 shows in an exaggerated way, how the contact elements 45 on the ribs 37 elastically depress the areas 19 of the second electrically conductive coating 18 of the sensor element 10, thereby creating a sufficient contact pressure between the contact elements and the electrically conductive coating 18. The conductive ink of the engagement portion 30 permits good X-ray translucency of the engagement portion 30 in the same way as explained above.

In order to keep the conductors 44 in position during insertion moulding of the cable 7 in the engagement portion 30, protrutions are located in the mould used, above the locations where the apertures 43 are to be formed in the engagement portion, whereby pointed notches 46 are left in the top side 34 of the engagement portion.

The cable 7 extends from the cable connector 2 in a direction forming an angle β of approximately 15 degrees with the direction of the second side 5 of the electrode body 3. The direction of the second side 5 of the electrode body 3 corresponds to the direction indicated with the broken line 24 in Fig. 18, in the connected position of the cable connector on the electrode. This arrangement ensures that a slim configuration of the electrode body and connector is obtained, whereby the cable may extend close to the second side 5 of the electrode body.

In order to improve the X-ray translucency and/or improve the securing of the conductors 44 of the cable 7 in the cable connector, the conductors 44 may be spread out to the form of a fan before they are embedded in the engagement portion 30. Further, the X-ray translucency may be improved by employing cable conductors 44 made of carbon.

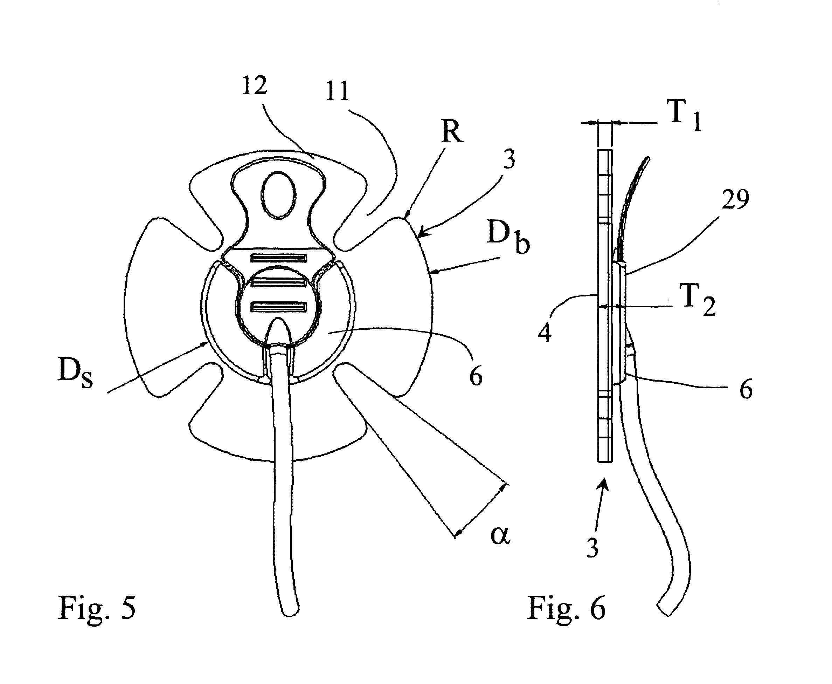

Referring to Fig. 5 and 6, advantageous dimensions of a neonatal skin electrode according to the invention are as follows: a thickness of the electrode body T1 = 0.8 millimetres, and a thickness between the first side 4 of the electrode body and the top side 29 of the socket 6, T2 = 0.6 millimetres, an outer peripheral diameter of the electrode body 3, D_{b} = 18 millimetres, an outer peripheral diameter of the socket 6, Dₛ = 9 millimetres, a radius of curvature connecting the cutouts 11 and the peripheral contour of the electrode body 3, R = 0.8 millimetres, and an angle between two longitudinal opposed sides of the cutouts 11, α = 10 degrees. Cutouts 11 having approximately dimensions as mentioned above and a configuration as shown in the Figures are particularly advantageous in order to enable the electrode body 3 to adapt its shape to the skin of the patient, in particular a neonatal, as it adapts well to even very irregular and small parts of the skin. The peripheral contour and in particular the cutouts 11 of the electrode body 3 is an invention in itself and may be applied to other types of electrodes having other types of connector arrangements than the arrangement described in this application. Although the indicated dimensions may be advantageous, the electrode arrangement according to the invention may equally have other suitable dimensions without leaving the scope of the invention.

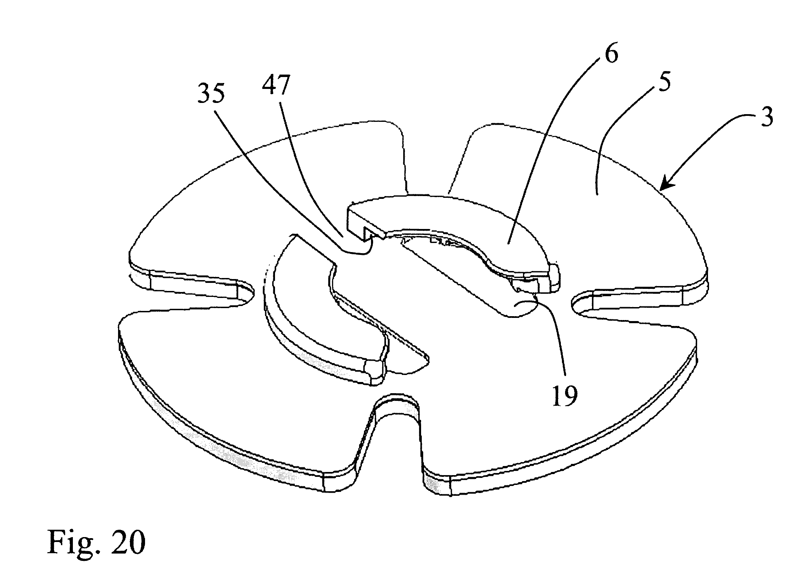

Fig. 20 shows another embodiment of the electrode 1, whereby the socket 6 is split up in two separate opposed parts, thereby creating a space 47 that enables the cable 7 to extend from the cable connector 2 close to the second side 5 of the electrode body 3. The socket 6 may similarly be split up in more separate parts being mutually spaced.

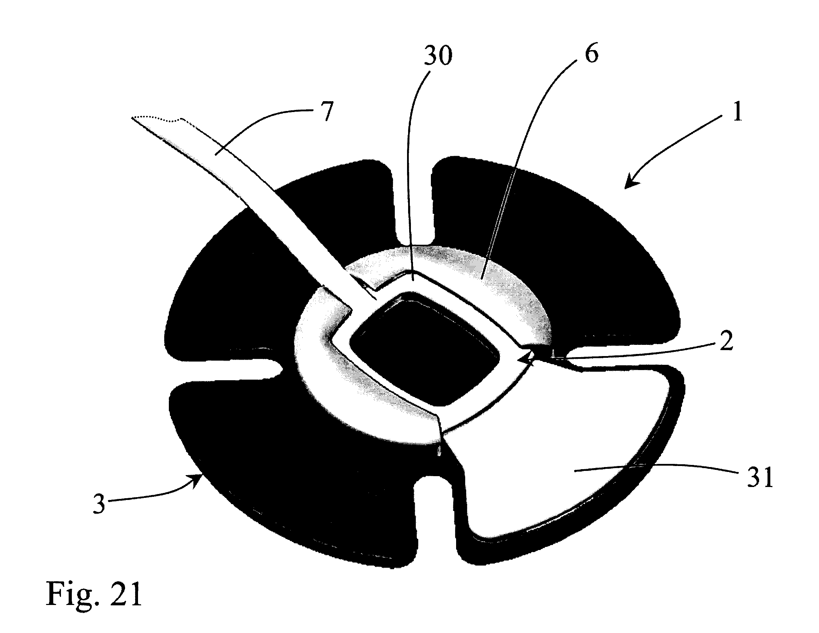

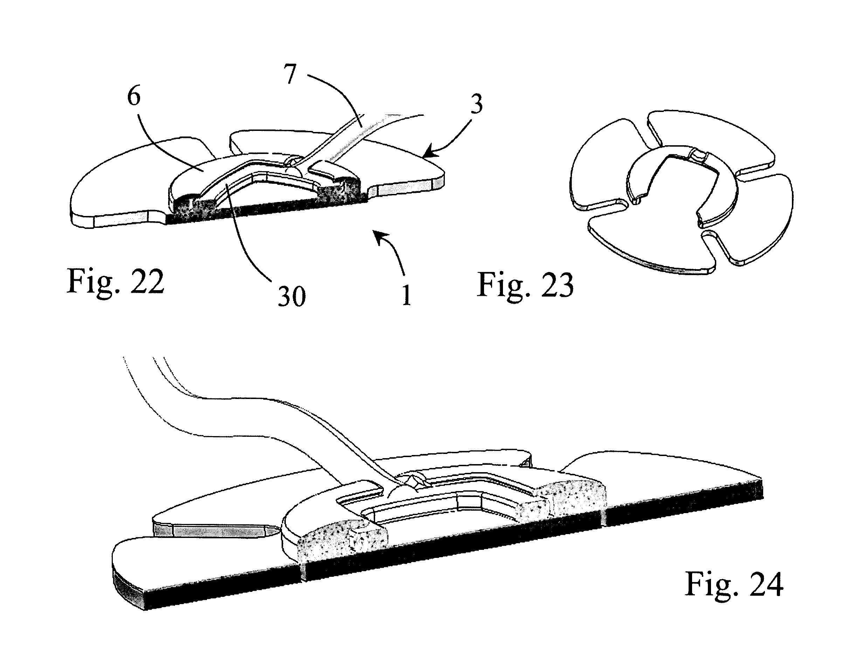

Figures 21 to 24 show another embodiment of the electrode 1 and cable connector 2 according to the invention. In this embodiment, both the socket 6 and the engagement portion 30 of the cable connector 2 are made of electrically conductive plastic, such as polypropylene comprising carbon fibres or any other similar suitable material, in order to provide the electrical connection between the cable 7 and the electrically conductive gel 8. The engagement portion 30 has substantially the shape of a rectangular ring composed of two longitudinal extending side members connected by two opposed end members. A central section of each side member is slightly curved away from the opposed side member in the disengaged position of the engagement portion, and when inserted into the socket 6 said central sections are deflected in a direction towards each other, whereby a frictional force between the engagement portion 30 and the socket 6 is achieved, whereby the engagement portion is kept in place.

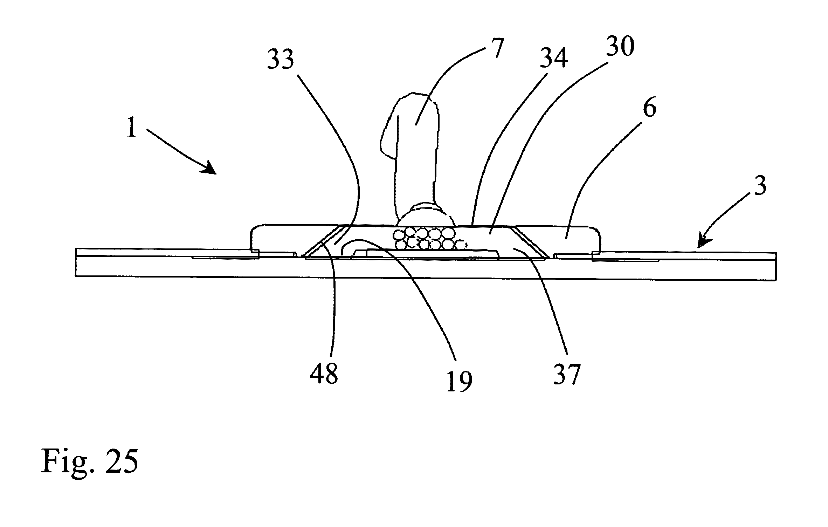

Fig. 25 shows another embodiment of the electrode, whereby the engagement portion 30 has two opposed edge portions 33 which are depressed in relation to the top side 34 in an inclined manner, and the two opposed groove sections 35 opening against each other in the socket 6 have respective inclined top faces 48 corresponding to the inclined edge portions 33.

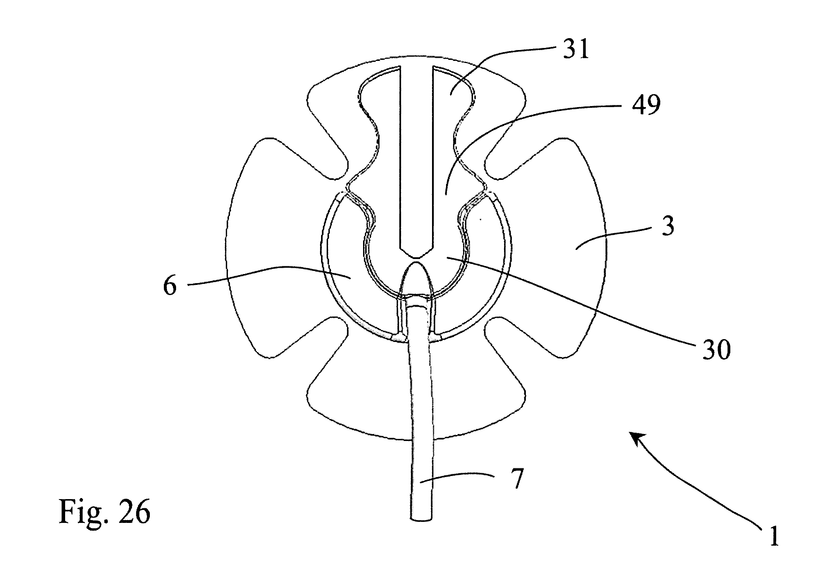

Fig. 26 shows another embodiment of the electrode, whereby the grip portion 31 and a substantial part of the engagement portion 30 of the cable connector 2 are split up longitudinally in to halves 49, so that these may be pressed elastically against each other at the insertion of the engagement portion 30 into the socket 6. In the inserted position, the two halves 49 of the engagement portion 30 will flex elastically back and abut the respective two opposed flange members of the socket 6, thereby exerting a contact pressure against the socket 6.

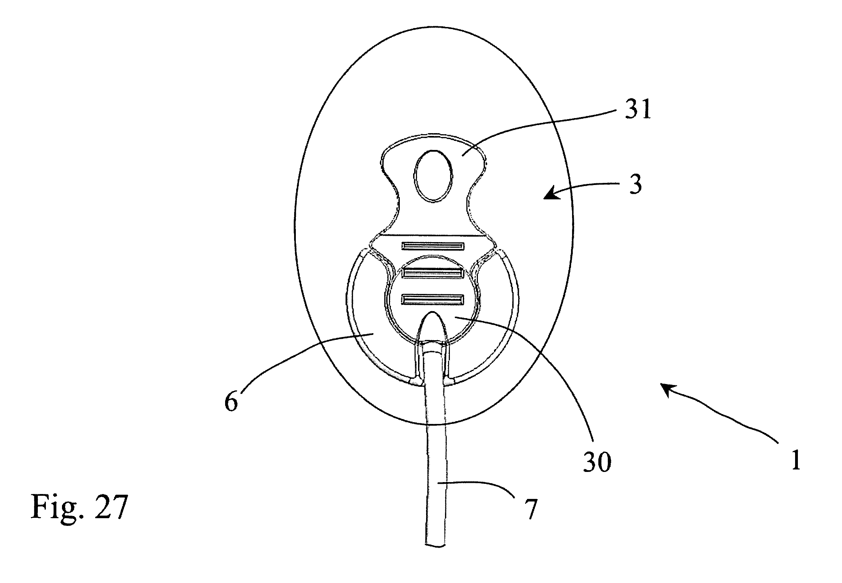

Fig. 27 shows yet another embodiment of the electrode 1, whereby the electrode body 3 has an oval contour without any cuttings, and the socket 6 is offset from the central part of the skin contact establishing portion of the electrode body 3. Apart from these differences, this embodiment corresponds to the electrode previously discussed, so that the socket 6 and the cable connector 2 may have any of the configurations shown in the other Figures.

Other embodiments than those shown in the Figures are possible within the scope of the invention; for instance, the engagement portion 30 of the cable connector 2 may be provided with only one central rib 37 instead of two ribs, or it may be provided with more than two ribs, whereby in such cases a corresponding number of areas 19 of the second electrically conductive coating 18 may be provided on the sensor element 10, or alternatively one integrated area 19 of coating corresponding to all the ribs may be provided.

The above discussed embodiments may be combined in a number of different ways, for instance the gripping portion shown in Fig. 21 may be employed on the electrode 1 in Fig. 1, the split up engagement portion 30 in Fig. 26 may be combined with the inclined edge portions 33 in Fig. 25, as well as with the ringshaped engagement portion 30 in Fig. 21 or the split up socket in Fig. 20, and so on.

## Claims

1. An electromedical electrode with a detachable cable connector, comprising an electrode body having a first side adapted to contact the skin of a patient and a second side opposite to the first side, at least one flange member extending along the second side of the electrode body and forming at least two opposed groove sections, the cable connector having two opposed edge portions slidable into the respective groove sections of the at least one flange member, whereby the cable connector may be retained in a connected position on the electrode, **characterized in that** the electrode body has a substantially thin configuration, the cable connector has a substantially flat engagement portion, the at least two opposed groove sections open towards each other, whereby the engagement portion of the cable connector, in the connected position of the cable connector on the electrode, is at least partly encircled by the at least one flange member.

2. An electromedical electrode according to claim 1, **characterized in that** a cable extends from the cable connector in such a direction that, by pulling the cable, the engagement portion may be slid in a mounting direction into the groove sections of the at least one flange member, until it reaches its connected position on the electrode, and **in that**, in its connected position, the engagement portion is prevented by means of the flange member from sliding further in the mounting direction.

3. An electromedical electrode according to claim 1 or 2, **characterized in that**, in the connected position of the cable connector on the electrode, a cable extends from the cable connector in a direction forming an angle with the second side of the electrode body of at least 8 degrees, and preferably at least 12 degrees.

4. An electromedical electrode according to any one of the preceding claims, **characterized in that**, in the connected position of the cable connector on the electrode, a free top side of the engagement portion of the cable connector is substantially flush with a free top side of the at least one flange member.

5. An electromedical electrode according to any one of the preceding claims, **characterized in that** the two opposed edge portions of the cable connector are depressed in relation to the free top side of the engagement portion of the cable connector.

6. An electromedical electrode according to any one of the preceding claims, **characterized in that** the at least one flange member is constituted by a socket having substantially the shape of part of a ring, the at least two opposed groove sections being formed by opposed parts of the socket, respectively.

7. An electromedical electrode according to claim 6, **characterized in that** the socket has substantially the shape of a circular section of at least 180 degrees, preferably at least 200 degrees, and in particular at least 220 degrees of a ring.

8. An electromedical electrode according to any one of the claims 6 or 7, **characterized in that** the groove sections have a substantially U-shaped cross-section delimited partly by a channel formed in the socket and having a substantially L-shaped cross-section and partly by the second side of the electrode body.

9. An electromedical electrode according to any one of the claims 6 to 8, **characterized in that** the at least two groove sections are arranged symmetrically on either side of a projection formed integrally in the socket and separating the groove sections from each other, and **in that** an indentation is formed between the two opposed edge portions of the cable connector, the indentation receiving the projection of the socket in the connected position of the cable connector on the electrode.

10. An electromedical electrode according to any one of the claims 6 to 9, **characterized in that** each of the two opposed edge portions of the cable connector has at least one notch in which a tooth formed in the channel in the socket may engage elastically, when the cable connector is inserted in the socket.

11. An electromedical electrode according to any one of the claims 6 to 10, **characterized in that** a recess is formed in the free top side of the socket opposite the projection that separates the groove sections in the socket, and **in that** the recess, in the connected position of the cable connector on the electrode, receives part of a cable extending from the cable connector.

12. An electromedical electrode according to any one of the claims 6 to 11, **characterized in that** an electrically conductive sensor element of the electrode body is exposed on the second side of the electrode body, and **in that** at least one contact element of the cable connector contacts the sensor element in the connected position of the cable connector on the electrode.

13. An electromedical electrode according to claim 12, **characterized in that** the sensor element is sandwiched between an electrically nonconductive electrode foil and an electrically conductive and adhesive gel, the nonconductive electrode foil having, in an area encircled at least partly by the socket, at least one aperture corresponding to the at least one contact element of the cable connector.

14. An electromedical electrode according to claim 13, **characterized in that** the at least one aperture in the nonconductive electrode foil comprises two opposed elongated apertures, each extending along a respective one of the two opposed groove sections formed by the socket.

15. An electromedical electrode according to any one of the claims 13 or 14, **characterized in that** the electrically conductive sensor element is formed by an electrically nonconductive sensor foil having a first side with a first electrically conductive coating contacting the electrically conductive and adhesive gel and a second side with a second electrically conductive coating, the first electrically conductive coating covering substantially the entire area of the first side of the nonconductive sensor foil, and the second electrically conductive coating covering the areas of the conductive sensor element exposed through the at least one aperture in the nonconductive electrode foil.

16. An electromedical electrode according to claim 15, **characterized in that** the electrically conductive sensor element has an edge region extending along the entire peripheral edge of the sensor foil, whereby said edge region is not covered by the first and second electrically conductive coatings, and **in that** a part of said edge region is coated with conductive ink.

17. An electromedical electrode according to claim 16, **characterized in that** the first electrically conductive coating comprises silver and silver chloride, the second electrically conductive coating comprises silver and the conductive ink comprises carbon.

18. An electromedical electrode according to any one of the claims 12 to 17, **characterized in that** the sensor element is arranged at a central part of the electrode body.

19. An electromedical electrode according to any one of the claims 12 to 18, **characterized in that** the socket has legs extending through corresponding holes in the electrically nonconductive electrode foil and the sensor element, an end of each leg having expanded cross-section and thereby securing the socket on the electrode body.

20. An electromedical electrode according to claim 19, **characterized in that** the socket and its legs are integral, and **in that** the expanded cross-section of each leg is formed by heat-melting the plastic material of the leg.

21. An electromedical electrode according to any one of the preceding claims, **characterized in that** the cable is insert moulded in the engagement portion of the cable connector, **in that** at least one cavity is formed in a bottom side of the cable connector, whereby a conductor of the cable is exposed in the cavity, and **in that** the at least one contact element of the cable connector is formed by a coating of conductive ink extending to the exposed cable conductor and thereby establishing electrical connection to the latter.

22. An electromedical electrode according to claim 21, **characterized in that** a pointed notch is formed in the top side of the engagement portion opposite each cavity in the bottom side of the engagement portion.

23. An electromedical electrode according to any one of the claims 1 to 20, **characterized in that** the engagement portion of the cable connector comprises two corresponding parts which are joined together so that a cable end is secured between said two parts.

24. An electromedical electrode according to any one of the preceding claims, **characterized in that** the engagement portion of the cable connector has the form of a ring adapted to deform elastically, when inserted into the at least one flange member, thereby providing a frictional force between the ring and the flange member.

25. An electromedical electrode according to claim 24, **characterized in that** the ring has a substantially rectangular shape having two opposed long sides constituting the edge portions slidable into the respective groove sections of the at least one flange member, and **in that** said edge portions are slightly curved symmetrically in relation to each other.

26. An electromedical electrode according to any one of the preceding claims, **characterized in that** the engagement portion of the cable connector and the at least one flange member on the electrode body are made of electrically conductive plastic, such as polypropylene comprising carbon fibres, and **in that** preferably at least the top side of the engagement portion and the top side of the cable connector are provided with an electrically nonconductive coating.

27. An electromedical electrode according to any one of the preceding claims, **characterized in that** the engagement portion of the cable connector is formed integrally with a slim grip portion extending opposite the cable, the grip portion bending slightly upward in relation to the top side of the engagement portion and preferably being perforated by a cutout.

28. An electromedical electrode according to any one of the preceding claims, **characterized in that** the electrode body has a substantially circular periphery provided with two or more radially extending cuttings, whereby each cutting, along substantially its entire length, has a minimum width of at least 5 per cent, and preferably at least 6 per cent, of the diameter of the electrode body, and **in that** the at least one flange member is arranged centrally on the electrode body.

29. An electromedical electrode according to claim 28, **characterized in that** each cutting has opposed side edges forming an angle of at least 6 degrees, preferably at least 8 degrees, and more preferably at least 9 degrees, **in that** these side edges are joined by a circular arc and join the circular periphery of the electrode body via circular arcs.

30. An electromedical electrode according to any one of the preceding claims, **characterized in that** the general thickness of the electrode measured between the free top side of the at least one flange member and the first side of the electrode body is smaller than 3 millimetres, preferably smaller than 2 millimetres, and more preferably approximately 1.8 millimetres.
